(19)

Europäisches
Patentamt
European
Patent Office
Office européen
des brevets

(11) **EP 2 650 004 B1**

(12) **EUROPEAN PATENT SPECIFICATION**

(45) Date of publication and mention
of the grant of the patent:
**23.06.2021 Bulletin 2021/25**

(21) Application number: **11847420.4**

(22) Date of filing: **08.12.2011**

(51) Int Cl.:
*A61K 8/97* (2017.01)     *A61K 8/9789* (2017.01)
*A61K 36/22* (2006.01)     *A61P 17/16* (2006.01)
*A61Q 17/00* (2006.01)     *A61Q 17/04* (2006.01)

(86) International application number:
**PCT/MX2011/000149**

(87) International publication number:
**WO 2012/078018 (14.06.2012 Gazette 2012/24)**

(54) **PRODUCTION OF STANDARDIZED EXTRACTS OF THE PLANT KNOWN AS "CUACHALALATE" (AMPHIPTERYGIUM ADSTRINGENS) AND USES THEREOF AS SUN PROTECTION AGENTS**

HERSTELLUNG VON STANDARDISIERTEN EXTRAKTEN DER ALS "CUACHALALAT" (AMPHIPTERYGIUM ADSTRINGENS) BEKANNTEN PFLANZE UND DEREN VERWENDUNG ALS SONNENSCHUTZMITTEL

OBTENTION D'EXTRAITS NORMALISÉS DE LA PLANTE CONNUE SOUS LE NOM DE "CUACHALALATE" (AMPHIPTERYGIUM ADSTRINGENS) ET LEURS APPLICATIONS EN TANT QU'AGENTS DE PROTECTION SOLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2010 MX 2010013512**

(43) Date of publication of application:
**16.10.2013 Bulletin 2013/42**

(73) Proprietor: **Bioextracto S.A. De C.V.**
**C.P. 11800 México D.F. (MX)**

(72) Inventors:
- **MAS OLIVA, Jaime**
  **C.P. 01900 México D.F. (MX)**
- **HURTADO BOCANEGRA, Ma. Dolores**
  **C.P. 07469 México D.F. (MX)**

(74) Representative: **Clarke Modet & Co.**
**C/ Suero de Quiñones 34-36**
**28002 Madrid (ES)**

(56) References cited:
JP-A- 11 209 261      JP-A- 11 209 298
JP-A- H11 209 223     JP-A- 2000 198 715
JP-A- 2004 307 443    JP-A- 2006 062 990
US-A- 5 843 421

- **DATABASE WPI Week 199941 Thomson Scientific, London, GB; AN 1999-488741 XP002718204, & JP H11 209223 A (SHISEIDO CO LTD) 3 August 1999 (1999-08-03)**
- **DATABASE KOSMET [Online] GANEM RONDERO, F. A. ET AL.: 'Study of the photoprotective-cosmetic properties of Amphipterygium adstringens (Cuachalalate) extract', XP055098572 Retrieved from STN Database accession no. 16845 & IFSCC INTERNATIONAL CONFERENCE, THE COSMETIC IMAGE : A MOSAIC OF BIOSCIENCES vol. 3, October 1994, pages 686 - 703**
- **CÉDRIC SAUCIER ET AL: "Characterization of (+)-Catechin-Acetaldehyde Polymers: A Model for Colloidal State of Wine Polyphenols", JOURNAL OF AGRICULTURAL AND FOOD CHEMISTRY, vol. 45, no. 4, 1 March 1997 (1997-03-01), - 4 April 1997 (1997-04-04), pages 1045-1049, XP055696570, US ISSN: 0021-8561, DOI: 10.1021/jf960597v**
- **Mariana Von Staszewski ET AL: "Influence of green tea polyphenols on the colloidal stability and gelation of WPC", Food Hydrocolloids, vol. 25, no. 5, 1 July 2011 (2011-07-01), pages 1077-1084, XP055696572, NL ISSN: 0268-005X, DOI: 10.1016/j.foodhyd.2010.10.004**

**(Cont. next page)**

- **Mahmut Özacar ET AL: "Evaluation of tannin biopolymer as a coagulant aid for coagulation of colloidal particles", COLLOIDS AND SURFACES A: PHYSIOCHEMICAL AND ENGINEERING ASPECTS, vol. 229, no. 1-3, 1 November 2003 (2003-11-01), pages 85-96, XP055696575, AMSTERDAM, NL ISSN: 0927-7757, DOI: 10.1016/j.colsurfa.2003.07.006**

**Description**

**FIELD OF INVENTION**

The present invention refers to production of plant extracts for their use in the field of sun protection, particularly for producing standardized extracts of the Mexican plant known as Cuachalalate *(Amphipterygium Adstringens)* and more particularly for producing standardized extracts from *Amphipterygium Adstringens* bark and leaves in order to produce a useful phytocomplex both for prevention and treatment of induced skin lesions due to an overexposure to sun radiation.

**BACKGROUND OF INVENTION**

The sun is the main source of ultraviolet (UV) radiation exposure for most individuals. Sunlight comprises visible light, infrared and ultraviolet radiation. UV radiation is part of the electromagnetic spectrum non-ionizing energy which wavelength oscillates between 100 and 400 nm and constitutes the electromagnetic spectrum most energetic portion that affects earth surface. UV radiation is conventionally classified in three different regions: UVA (>315-400 nm), UVB (>280-315 nm) and UVC (>100-280 nm) ; in this classification, UVC radiation is mostly absorbed by the atmospheric ozone while most of UVA radiation and about 10% of UVB reach earth surface. Therefore, UVA and UVB radiation are of most importance for human health and they are clinically relevant as they contribute to several skin pathologic alterations (WHO, 2009; IARC, 2005).

Sunlight deleterious effects over skin are well-documented. However, people are forced to stay below the sun during long terms because of their work. Others are overexposed to sun during leisure time activities and/or when wishing a tanned appearance observed after sun radiation and/or artificial light exposure with wavelengths between about 290 and 400 nm.

The main short-term noticeable effect of UV radiation overexposure is induced by wavelengths between 280 and 315 nm (UVB region) of sufficient intensity, inducing a number of pathological changes in skin including erythema appearance (typical burn characterized by skin redness), edema, blister formation or other skin outbreaks.

There are other long-term effects attributed to a chronic or excessive radiation exposure at these wavelengths, such as photoaging or skin cancer, in addition to the short-term erythema risk caused by sunlight. Photoaging, or early skin aging, is characterized by skin wrinkling and yellowing together with other physical changes such as chapping, elasticity loss, teleangiectasis (arachnoid vessels), ecchymosis (subcutaneous hemorrhagic lesions) and malign skin surface growths. Concerns about UV radiation with wavelength higher than 315 nm (UVA region) and its effects on human skin have recently increased as some studies show that it is not only deleterious for skin but also has negative effects on eyes and even on immune system (Ley R, 1997).

Solar UV radiation exposure is currently scientifically accepted to represent the most significant risk factor for skin cancer development. According to World Health Organization (WHO) between 50% and 90% of skin cancers are caused by exposure to UV radiation, whether solar radiation or from artificial sources such as tanning beds (WHO, 2009). More than 200,000 melanoma cases were diagnosed worldwide in 2000 and 65,000 melanoma-associated deaths were reported. On the other hand, there are about 2.8 million cases of squamous cell carcinoma (CCE) and 10 million cases of basal cell carcinoma (CCB); these two types are the largest incidence cancer worldwide and are collectively called non-melanoma type skin cancer (WHO, 2009; AAD, 2008). CCE and CCB are the two most significant non-melanoma type skin cancer manifestations. CCB is the most common skin cancer form with about one million cases diagnosed every year and although rarely fatal, it may be disfiguring. On the other hand, although CCE incidence is lower, the mortality index is higher and about 2,500 associated deaths are annually diagnosed out of 250,000 cases (NCI, 2007).

However, human being needs of some amounts of solar exposure in spite of the induced damages. It is well known that small UV radiation amounts are essential for vitamin D production. When skin is exposed to sunlight, UVB radiation is absorbed in epidermis and dermis by 7-dehydrocholesterol and then converted into previtamin $D_3$ which once formed, is rapidly induced to isomerization because of body temperature and then converted into vitamin $D_3$ (WHO, 2009; Holick MF, 2008). There is a debate up to now about how much solar exposure is suitable to provide a proper balance between UV radiation positive and negative effects, such as skin cancer induction and vitamin D status into organism (Reichrath J, 2009) .

Although immediate UV radiation effects may be cosmetically and socially justified, even for physical wellness causes, long-term effects are cumulative and potentially hazardous; thus, protection against UV radiation is critical for preventing these and other deleterious effects as that is considered the main etiologic agent in most of skin cancer types as well as a key factor in photoaging.

Photoprotection involves all those methods used to prevent the damage caused in skin by UV radiation; including sun screens, physical barriers between skin and solar radiation such as clothing, hats and sunglasses, and searching for shadow and a proper hour and period for daylight exposure (Kaminester LH, 1996; Wang SQ, et al., 2009). Sun screens are found among the best photoprotective measures against UV radiation and growth in the field of sun screen products has been exponential because of an awareness of the risks associated with solar exposure.

Development of sun screens has been performed from a beginning in parallel with a sun tanning trend in the 30's, when those were designed for blocking UVB rays in order to prevent sun burns. Along the 80's, a number of epidemiological studies demonstrated a strong association between sun-induced burns and skin cancer at the time that several studies in animals showed that solar filters may also reduce a great number of UV radiation induced skin lesions, such as solar keratosis and CCE, in addition to delaying erythemal reaction (Autier P, 2009; Autier P, et al., 2007)

A number of protective agents capable of filtering UV rays have been developed in recent years and used in commercial formulations. There are opaque materials such as zinc oxide and titanium dioxide which literally provide a physical barrier for sun rays. On the other hand, there are chemical agents absorbing UV radiation such as cinnamates, amino benzoic acid derivatives, benzophenones, anthranilates, and homomenthyl salicylate.

Topical sun screens are comprised of chemical or physical agents that provide protection against UV radiation when applied onto skin. They are designed for sun UV radiation blockage and/or absorption, their use has potential for decreasing the skin cancer risk and for delaying the photoaging process (Wang SQ, et al., 2009).

Several plant derivative extracts or products have been observed to stimulate epithelial restoration and various conditions, such as injuries and ulcers of different etiology, including lesions commonly associated with solar overexposure. Thus, multiple plant derivative treatment options have been developed such as the use of aloe biologically active polysaccharides, herbal powdered materials for treatment of eczema and psoriasis, such as those obtained from Neem tree bark *(Melia azadirachta)* and tepescohuite *(Mimosa tenuiflora)*. On this regard, a very old use plant, cuachalalate *(Amphipterygium adstringens),* confirmed as anti-cancer, anti-malaria, astringent and for typhoid fever within a broad spectrum of biological activities, as well as noticeable hypocholesterolemic and antiulcer effects, is also frequently used for treatment of skin ulcers and lesions. As to its use for skin lesions (including acne), the cuachalalate is still used in a rudimentary way through a propagation of ground bark derived powder over the wound, or by locally washing with a boiled preparation thereof, neither allowing a proper use of its active ingredients nor a suitable active dosage. On this regard, the use of cuachalalate in the field of skin care is very common by applying it over pimples, ulcers and sores in humans and animals, further used in relieving baby skin rubbing and also used in blows, bites and stings and for wound healing.

Cuachalalate extracts are hydrosoluble or hydrophyllic extracts that may be obtained by alcohol or polar solvent standard extractions from leaves, bark or rhizome. Polar extraction may be followed by a filtering and concentration process. Additional steps may be used for adding or withdrawing components. An example of general protocols for producing *Amphipterygium adstringens* polar extracts may be found in US5843421.

As used below, "EC" abbreviation means "Cuachalalate extract" which is extracted from cuachalalate *(Amphipterygium adstringens)* bark and leaves and having photoprotective, antioxidant and skin-regenerative properties.

Natural remedies in case of photoprotection, mainly for "aftersun" palliative care, may be used as nature provides a number of plants which phytochemical profile includes analgesic, antiseptic, antioxidant, moisturizing, emollient activity and a remarkable ultraviolet radiation absorption activity.

*Amphipterygium adstringens* Schiede ex Schiecht (Julianaceae Family) is a medicinal tree known as "cuachalalate" widely used in traditional medicine throughout Mexico.

*Amphipterygium adstringens,* is a tree about 8 m high, which wood is used since pre-Hispanic times to treat cancer, gastric ulcer, blood purification, wound healing, bleeding, sores and anemia. This species of usually twisted stem with few branches, a smooth bark with large thickened and suberificated scales; bonded male flowers in panicles up to 15 cm long, single female flowers in the leaf axils, blooms from May to July in warm-dry weather and loses its leaves between October and June, the fruit is a dry one-seeded fibrous fruit with reddish-brown color. The plant usually grows in deciduous forests in the states of Jalisco, Colima, Guerrero, Mexico, Puebla, Nayarit, Morelos, Michoacan, being mainly distributed in the Balsas basin and southeastern Oaxaca. The species regularly exists in hot, semitropical and temperate climates.

Cuachalalate *(Amphipterygium adstringens)* is generally used for the treatment of colitis, fevers, recent wounds, hypercholesterolemia, gastritis, gastric ulcers, gastrointestinal cancer and various inflammatory diseases. Pharmacological studies in this species have demonstrated that hexanol and methanol extracts exhibit anti-ulcer activity and hypocholesterolemic activity, respectively.

The presence of masticadienonic, instipolynacic, cuachalalic, 3-epi-masticadienoic, 3 alpha-hydroxy-masticadienonic, isomasticadienoic and epi-oleanolic acids, β-sitosterol and three alkyl anacardic acids has been identified in the stem bark. Benzyl compounds of 6-heptadecyl-, 6-nonadecyl-, and 6-pentadecyl salicylic acids, and a mixture of phenolic aldehydes have also been isolated.

Phenolic compounds are secondary metabolites that comprise a variety of molecules of various structures such as cinnamic acids, benzoic acids, proanthocyanidins, coumarins and flavonoids. Most of polyphenols can absorb UV radiation such that, when applied topically, may prevent radiation penetration into skin by acting as natural sunscreens, helping to reduce the damage induced by UV radiation in the skin.

A variety of polyphenols, mostly included in diet, have shown photoprotective effects on the skin. Studies in animals have shown that polyphenolic compound oral or topical application, improves the adverse skin reactions that occur due to UV radiation overexposure, including erythema, skin lesions and lipid peroxidation. For example, it has been observed that an isolated polyphenolic fraction from green tea has multiple activities associated with prevention in animal models and in vitro systems. Previous studies in mice have shown that topical application of polyphenols prior to UV exposure provided significant protection against erythema, skin lesions, skin cancer and immunosuppression induced by UVB radiation (280-315nm).

Thanks to the evidence provided by these and other studies, polyphenols have been suggested for favorably supplementing sunscreen products and these may be useful in preventing skin diseases associated with inflammation and oxidative stress induced by UV solar radiation exposure.

On the other hand, since a number of compositions using or comprising *Amphipterygium adstringens* extracts have shown favorable effects in treatment of cutaneous lesions, one of their main uses is in commercial preparations for treatment of human acne and other dermatoses.

In spite of a great amount of available agents, there is a field of application in searching new and better agents with capability of filtering ultraviolet radiation. One of the main causes driving this search is a continuous demand of "natural" products. Many agents found in nature are capable of performing the same or any equivalent function to that of chemical synthesis compounds, however, provided protection level has been noticed not to allow a long sun exposure.

Thus, there is a continuous demand of natural agents in solar filters being as effective as currently available synthetic agents. In the field of sun protection, plant extracts including compounds capable of selectively absorbing certain UV spectrum wavelengths have been found, particularly in the zone between 280 and 400 nm corresponding to UVA and UVB radiation. These complex natural mixtures are more frequently used worldwide as said extracts are capable of several other cosmetic functions largely due to a variety of molecules present therein, which in addition to providing said features they act synergistically providing better results.

Prior art uses solar filter agents applied on skin for protection against erythema caused by radiation and for providing the exposure degree delivered by a safe tanning. Said agents are varied and mostly comprise chemical synthesis compounds such as para-aminobenzoates, benzophenones, cinnamates, salicylates, gallates and mixtures thereof.

A number of plant extracts have been used in the field of photoprotection for preparing sun burn palliative products (aftersun products) such as marigold, lettuce, apricot, cucumber, chamomile, and others; solar filters include walnut tree, rosemary, valerian, aloe vera -a favorite among formulators-, and others.

The skin is the most extensive and exposed human body tissue because of its direct interaction with environment. Consequently, skin lesions are the most commonly experienced events by human beings such that a person has suffered skin lesions and/or injuries and experienced their healing process at least once in a life. In spite of wound appearance and healing as relatively common facts, there are a number of factors which ease or worsen healing.

Wound healing involves a complex and arranged sequence of cell events ending in a restoration of damaged tissue structural integrity. The presence of cells which stimulate the inflammatory process and cell growth factor production, blood vessel in situ development and extracellular matrix production are important factors for a fast and efficient wound healing. Although these cell repair events allow a normal restoration of damaged tissues and their performance, there are a number of factors making a proper wound restoration difficult, causing for instance a continuity disruption in the outer epidermal layer and tissue loss with a consequent exposure of internal tissue thus creating chronic lesions not permitting to complete the healing process nor the epidermal tissue regeneration.

Cuachalalate is frequently used, mainly as a boiled preparation, for treatment of ulcers and certain skin lesions; ulcer treatment is joined with drinking an aqueous bark softened preparation in many states of Mexican Republic. Wounds are relieved by applying powder thereto or with a local washing with an aqueous bark softened preparation in addition of drinking the boiled preparation. On the other hand, the use over pimples and sores is carried out by drinking a boiled preparation and/or by application of bark resin or white gum. Human and animal wounds and sores are similarly treated with washings once a day together with a powdered application over the affected part, three times a day. Other known and recommended traditional uses include baby rubbings, blows, abscesses, poisonous animal bites and stings due to their antiseptic, anti-inflammatory and healing properties.

The use of cuachalalate in the traditional way over skin lesions of different etiology is carried out rudimentarily and hindering a full use of its actives; thus, this invention also refers to a method for obtaining a formulation which eases the use of a cuachalalate preparation as a therapeutic agent for treatment of human skin lesions.

Preparations including cuachalalate in their composition are currently used in capillary cosmetic preparations comprising triterpenes with a demonstrated inhibiting activity over testosterone 5-alpha reductase deemed relevant for its applications on human baldeness as disclosed in documents JP10245393, JP10245394, JP11246413, JP11246415, JP2000103765, US20040022758. On the other hand, another more patented activity in preparations including cuachalalate as the main ingredient(s) refers to the field of skin care, as agent in treatment of acne and other dermatoses JP10245393, JP10245394, JP11209236, JP11209298, JP11246413, JP2000169497. Moisturizing and stimulating properties are attributed in the field of skin care so that documents JP11209261, JP11209298 and JP2000247862 are deemed relevant in this field.

As noticed in above search, Cuachalalate is present in technological developments through patents or patent applications showing thus an interest in marketing natural ingredient based products and particularly Cuachalalate.

JP 2004 307443 teaches that hair loss is due to a caspase dependent apoptosis mechanism. Since caspase-9 is apparently inhibited by a so-called "herbal medicine" the document concludes that this "herbal medicine" can be used as a hair restorer. Similarly, US5843421A teaches about an extract from Cuachalalate as a hair tonic. A hair tonic and the restoration of hair are not linked to the treatment of skin due to sun overexposure and should not affect the present invention.

JP 2006 062990 teaches that extracts obtained from the plant genus *Ageratum* restore damage caused by UV radiation in epidermal keratinocytes. The extract is able to heal skin from UV damage and works as a moisturizer, cell activator or anti-inflammatory agent, but does not mention it use as a protection agent against the damage of UV radiation in the skin. *Juliana adstringens* is mentioned in a list of plants among many others, not showing any direct evidence of an extract of this plant. For the expert, it would have been an undue amount of work to test each of them and select *Juliana adstringens* among the many others for the obtention of a colloidal extract to be used in the prevention and treatment of induced skin lesions due to sun radiation.

JPH 11209223 teaches about moisturizers and skin external preparations formulated with Cuachalalate extracts. A moisturizer is not a colloidal extract. The document does not mention or suggest the obtention of a colloidal extract nor discloses protection upon UV radiation. The expert in the search of prevention and treatment of skin lesions would have not got to a colloidal extract of the plant starting from the moisturizers disclosed in JPH 11209223.

Ganem Rondero, 1994, although teaching that an extract of *Amphipterygium adstringens* shows absorption between 320-255 nm, it also identifies the absorption with the effect of phenolic compounds. The document does not report achieving a colloid, however. A long maceration time is important to obtain a colloidal solution. The document does not disclose or suggest any long-term maceration of the starting material, but the preparation of a final powder that must be further dissolved in a hydroalcoholic solution (20% w/w). The resulting solution only reaches a specific gravity of 0.988, meaning that few quantity of organic compounds is effectively extracted from the starting material, not being able to form a colloidal solution. Therefore, the expert in the search of increasing the effectivity of an extract of *A. adstringens* in the prevention and treatment of sun lesions due to overexposure would not consider the teachings of Rondero.

JP 2000 198715 teaches that an extract of *A. adstringens* presents an inhibitory effect on the formation of lipid peroxides. The document does not mention UV radiation or the protective effects of *A. adstringens'* extracts upon UV radiation. Starting from these teachings, the expert in the search of prevention and treatment of skin lesions would have not arrived to a colloidal extract from A. *adstringens.*

JPH 11209298 teaches about the use of an extract of Cuachalalate as a product to treat rough skin and improve a clinical condition such psoriasis. It is mention that this extract presents protease inhibitory activity. The document does not deal with UV protection, however, and does not disclose or suggest that an extract of Cuachalalate could be of serve for the prevention or treatment of skin lesions due to sun exposure. The expert would have not taken into consideration the teachings of this document in the preparation of an extract for the prevention or treatment of skin lesions due to overexposure to sun radiation.

JPH 11209261 teaches about the effect of a Cuachalalate extract on hyaluronic acid production, particularly enhancing the hyaluronic acid-producing ability of the skin to prevent skin aging. Again, this document does not mention UV radiation and does not suggest the protective effects of *Amphipterygium adstringens* extracts upon UV radiation. The expert would have had no reason to take into consideration these teachings in the preparation of an extract for the prevention or treatment of skin lesions due to overexposure to sun radiation.

## BRIEF DESCRIPTION OF DRAWINGS

Figure 1. Infrared (IR) spectrum of Cuachalalate *(Amphipterygium adstringens)* raw extract.

Figure 2. UV absorption spectrum of Cuachalalate *(Amphipterygium adstringens)* hydroalcoholic extract scanned in a 190-1100 nm range over a 0.0625% sample.

Figure 3. *Amphipterygium adstringens* extract-treated CHO cell viability percentage (1:10, 1:100, 1:1000, 1:10000 and 1:100000 dilutions) during 12 hours. Each bar represents the mean $\pm$ E.E. of a quadruplicate experiment. ANOVA non-exposure control normalized, followed by Bonferroni.

Figure 4. *Amphipterygium adstringens* extract-treated CHO cell viability percentage (1:10, 1:100, 1:1000, 1:10000 and 1:100000 dilutions) during 24 hours. Each bar represents the mean $\pm$ E.E. of a quadruplicate experiment. ANOVA non-exposure control normalized, followed by Bonferroni.

Figure 5. *Amphipterygium adstringens* extract-treated CHO cell viability percentage (1:5, 1:25, 1:125, 1:625 and 1:3125 dilutions) during 24 hours. Each bar represents the mean ± E.E. of a quadruplicate experiment. ANOVA non-exposure control normalized, followed by Bonferroni, **p<0.001.

Figure 6. *Amphipterygium adstringens* extract-treated CHO cell viability percentage (1:2, 1:4, 1:8, 1:16 and 1:32 dilutions) during 24 hours. Each bar represents the mean ± E.E. of a quadruplicate experiment. ANOVA non-exposure control normalized, followed by Bonferroni, **p<0.001.

## DESCRIPTION

The present invention refers to the use of a natural composition with UV radiation absorption capacity comprising a colloidal hydroalcoholic concentrate extract of *Amphipteryngium adstringens* bark and leaves and between 12 and 36% w/w of tannins measured as tannic acid concentration, in the prevention and treatment of induced skin lesions due to an overexposure to sun radiation.

A second aspect of the present invention is a pharmaceutical composition comprising said composition for use, a high molecular weight acrylic acid polymer as gelling agent, an extract solvent polymer, an alkaline neutralization agent and water, in a pharmaceutically acceptable vehicle, in a topical composition in the form of a solution, an emulsion, a gel, an ointment, a lotion, a balm, a cream, a paste or a dispersible solid.

The present invention presents a natural product meeting efficacy features as a solar filter and others which make it interesting for use in formulations for solar protection, sun burn prevention and treatment and for skin lesions.

Although virtually impossible to accurately establish a direct relationship between the biological effects of a plant extract with a single molecule or group of molecules present in such a complex mixture, components that are in a higher proportion may be correlated, with the corresponding reserves, and then conducting studies to determine such a correlation.

The mixture of compounds comprising the composition described in the present invention and defined as the active principles responsible for the activity, is mainly composed of polyphenols and condensed tannins, which activity as protective agents for plant origin specimens against the environment, has been previously reported (Kitamura et al, 2004; Hinneburg et al, 2005). Tannins, flavonoids and other polyphenolic compounds have demonstrated that they may be used as sunscreens because of their UV-radiation absorption, antioxidant, anti-inflammatory and even antiproliferative properties, making them ideal to prevent and even alleviate burns caused by sun overexposure.

The invention comprises a hydroalcoholic Cuachalalate *(Amphipteryngium adstringens)* extract that, given its proven safety, is expected to be incorporated in cosmetic formulations for skin protection from ultraviolet sun radiation excessive exposure and to be part of therapeutic preparations for treatment of human skin lesions.

The present invention relates to a natural origin extract comprising a mixture of agents having the capability to absorb ultraviolet radiation and conferring photoprotective activity to said extract. Said mixture comprises a natural origin extract which provides moisturizing and emollient properties that contribute to relief lesions induced by sun overexposure events in addition to being capable of absorbing UV radiation.

The present invention discloses an *Amphipteryngium adstringens* plant standardized extract for obtaining preparations with sunscreen capability, thus providing an alternative to chemical synthesis sunscreens in photoprotective products.

In this regard, the present application discloses how to provide efficient and inexpensive methods to obtain *Amphipteryngium adstringens* plant standardized extracts that retain therapeutic activity.

The present invention discloses a non-toxic *Amphipteryngium adstringens* plant standardized extract that may relief various symptoms accompanying lesions by solar radiation overexposure.

The present invention relates to an *Amphipteryngium adstringens* plant standardized extract through tannin content normalization for the treatment of skin lesions caused by solar radiation overexposure.

The present invention discloses an *Amphipteryngium adstringens* plant standardized extract to obtain preparations which may be used to prevent sun harmful effects through its UV radiation absorption properties, in addition to be used as an "aftersun" product to refresh skin.

The present invention discloses an *Amphipteryngium adstringens* plant standardized extract useful as the basis for phytodrug production for treating skin lesions related to UV radiation harmful effects, comprising an *Amphipteryngium adstringens* plant standardized extract, as a therapeutic agent.

*The Amphipteryngium adstringens* extract characterizing the present invention is useful to be included into preparations for treating skin lesions of different etiology, including events associated with solar radiation overexposure.

The combined effects produced by *Amphipteryngium adstringens* extract show it as a good choice for use as an adjuvant to traditional skin lesion treatments, such as with the application of common methods and hygiene, ointments or salves, and in most severe cases, bandage compressions in the affected area.

The extract disclosed herein, represents a naturally occurring product, which, due to its UV absorption characteristics, its properties described in the traditional medicine of our country and the absence of toxic effects, may be used as basis for the design of preventive and/or palliative or adjuvant treatments in the treatment of skin lesions associated with sun exposure events. Moreover, the extract concept as a standardized extract envisages potential therapeutic applications within a controlled dosage regime.

The extract comprising predetermined quantities of active ingredients (tannins) is standardized as tannic acid equivalents, with the quantitative determination of tannic acid molecule in the extract, for example by UV-visible spectroscopy, for this purpose.

The natural preparation of the present invention may be formulated in many ways. The components of the present invention may be dissolved in aqueous solutions and for the formulation of a finished product may be combined with any compatible cosmetic or pharmaceutical carrier, for example, solutions, colloidal dispersions, emulsions, suspensions, creams, lotions, gels, foams etc. In this regard, particularly useful formulations are recommended as sunscreen lotions, after-sun lotions, for use after bathing regardless of the presentation. The compound mixture is capable of forming the basis of any of these presentations due to its high mixing capacity.

A pharmaceutical preparation using the extract of the invention as a therapeutic agent may be prepared according to any of the conventional methods and procedures. Any formulation suitable for local administration may be used. Pharmaceutical compositions comprising the present invention may be formulated to provide fast, sustained or prolonged active ingredient release after administration, using any of the methods and/or techniques well known in the art.

Particularly, the pharmaceutical composition of the invention may be topically applied for a localized effect on skin lesions. The active ingredients contained in the standardized extract of the invention may be also administered as appropriate for topical use. These dosage forms include solutions, colloidal dispersions, emulsions (oil/water or water/oil), suspensions, powders, creams, lotions, gels, foams, mousses, sprays and similar presentations.

Also included are compositions for topical use including solutions, liposome suspensions, water/oil or oil/water emulsions, gels, salves, creams, ointments and the like.

In the case of emulsions and suspensions including the composition these may contain nonionic, zwitterionic, anionic or cationic type surfactants, commonly used in the formulation of drugs. However, water/oil lipophilic emulsions are preferred for topical use. Moreover, solutions comprising the extract of the invention may be also formulated as a gel by addition of gelling agents. Specifically for hydrogel preparation comprising the phytodrug of the invention, we wish to remark that said dosage form has many advantages as an adjuvant in the treatment of skin lesions, as it provides a moist environment suitable for the wound. In this case, the favorable humidity conditions enable that the phytodrug of the invention isolates the wound from outside, restricts the entry of pathogenic microorganisms and allows contact with extract actives, eliciting an ideal environment for epithelial restoration.

Since the extract of the present invention is not toxic, it may be safely administered to humans, including skin ulcers. It may be also used in combination with other traditional treatments, increasing the treatment efficacy.

The extract of the invention is similar to phytoproducts in that is normally used in the treatment of skin lesions with its antimicrobial, analgesic and healing effects, but differs from many of them by its lack of toxicity while preserving the above effects that collectively confer therapeutic efficacy and safety in addition to simplifying the treatment by reducing the use of various medications.

The method used to obtain the extract of the invention allows obtaining *Amphipteryngium adstringens* hydroalcoholic extracts useful for the treatment of skin lesions due to their known anti-microbial, moisturizing and healing properties.

The active ingredients used in the present invention are of diverse chemical nature; they are present in nature and have not been chemically modified.

A new formulation is provided comprising an *Amphipteryngium adstringens* extract obtained by extraction with a hydroalcoholic solvent mixture. The extracted mixture is obtained by maceration and comprises active ingredients in an effective concentration. Mixing these extracts exhibits a 280-315 nm UV absorption range, with a peak maximum absorption at 297 nm.

The concentration of extract components becomes suitable with the method used for the obtention of the composition of the present invention; however, it is possible to adjust their concentration by a volume adjustment and the manipulation of weight ratios, both in the concentrated extract and in the obtained compositions.

As described in Example 1, and only as an example, the EC may be obtained by hydroalcoholic extraction from *Amphipteryngium adstringens* leaves and bark. Other hydrophilic solvents may be used in place of ethanol (for example, methanol, water or other alcohols). Multiple extractions are possible to perform after extraction in order to refine the polar or hydrophilic fraction, a number of fractionation and separation techniques may be employed (for instance, chromatography, dialysis, filtration, etc.) in various orders and multiplicities for extracting and isolating the active compound(s). After volatile solvent evaporation, the extract is typically syrupy in consistency and brown in color. Both color and consistency characteristics may vary depending on whether or not the extract is diluted with water or other solvents.

## EXAMPLE 1

The following describes the steps comprising the procedure for obtaining *Amphipteryngium adstringens* extract.

Step 1. It comprises obtaining the raw material from which the extracts are obtained. The obtained plant material is dehydrated and corresponds to the bark.

Step 2. It comprises a manual and visual selection of dehydrated bark, cleaning and removal of foreign material and heavy plant material.

Step 3. It comprises macerating the plant materials. The maceration process involves placing the plant material cut into pieces between 3 and 5 cm, in a container with a 50:50 alcohol:demineralized water solution. Once homogenized the mixture is allowed to macerate for a period from 12 to 15 days.

Step 4. It comprises macerate filtration by decantation with a mesh and cloth filter in order to remove all the plant material.

Step 5. It comprises bagasse separation from filter once the liquid has been removed.

Step 6. It comprises the macerate concentration. Once the solution is extracted from all the mixture components, then the solution resulting from the macerate filtration is poured into a rotary evaporator operating under the following conditions: bath at a 60°C temperature and 6 lnHg vacuum pressure.

Step 7. It comprises the collection and storage of the concentrate obtained in sun-protected containers and at room temperature.

The composition is described referred to the production method of Cuachalalate concentrate. The cuachalalate composition comprises the following ingredients: Cuachalalate, scientific name *Amphipterygium adstringens* in amount of 200-300 g per kilogram.

## II. Characterization of obtained EC.

Example 2 intends to illustrate the physicochemical characterization of *Amphipteryngium adstringens* extract obtained by above described method.

## EXAMPLE 2

The hydroalcoholic *Amphipteryngium adstringens* (Cuachalalate) extract shows an intense brown coloration with a sweet particular odor. The extract has an excellent solubility in methanol, is partially soluble in ethanol and water, slightly soluble in isopropanol, and practically insoluble in acetone and non-polar solvents. The solubility markedly increases in water/alcohol solutions with at least 50% alcohol. It shows a specific gravity of 1.034 and a pH of 5.2 in hydroalcoholic solution at 50% (w/w).

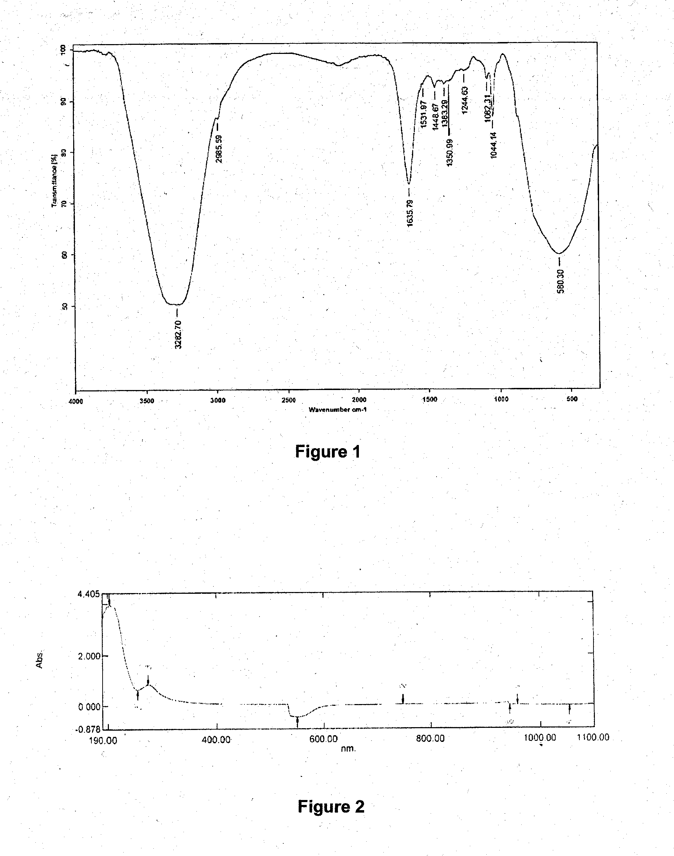

On the other hand, and continuing with the extract characterization, Figure 1 is included which corresponds to an infrared spectrum (IR), wherein the region strip profile known as the "fingerprint" being typical of polyphenolic compounds such as lignins, flavonoids and concentrated tannins is observed.

## III. Determination of EC absorption spectrum.

Ultraviolet radiation is subdivided into three wavelength regions. These regions are commonly referred to as UVA, UVB and UVC. UVA region extends from approximately 315 to 400 nm, UVB from 280 to 315 nm and UVC from 100 to 280 nm. Such UVC radiation is mostly absorbed by atmospheric ozone, while most of UVA and about 10% of UVB radiation reach the Earth's surface. Therefore, UVA and UVB are the most clinically important as relevant to human health, contributing to various skin pathological changes.

Example 3 illustrates the ability of *Amphipteryngium adstringens* extract for absorbing radiation in the UV range, obtained with the above described method.

## EXAMPLE 3

When an absorption spectrum of a composition shows specific absorption in the ultraviolet region, and particularly with peak absorption between 290 and 320 nm, said composition may be considered as a photoprotectant against sunburn or erythema, which is usually induced by solar radiation wavelengths between 290 and 320 nm.

The performed analysis consisted of conducting 190 to 1100 nm scans to determine the ability of *Amphipteryngium adstringens* hydroalcoholic extract to absorb radiation in the UV spectrum, at 3 different concentrations, using extract dilutions (0.25%; 0.125% and 0.0625%). Used extract was shown to absorb radiation in the range between 190 and 300 nm, corresponding to UVB. Therefore, such a composition may be included in preparations for prevention and handling of sun exposure harmful effects.

Figure 2 shows the absorption spectrum of the hydroalcoholic extract obtained by performing a scan in the range between 190-1100 nm. This extract represents the absorption capacity of the compound mixture comprising *Amphipteryngium adstringens* extract at a concentration of 0.0625%, herein described.

**IV. Determination of EC toxicity.**

**EXAMPLE 4**

This example illustrates the safety of *Amphipteryngium adstringens* extract, obtained with the method described above by assessing its toxicity in vitro. A determination of the extract cytotoxic effect prepared from the *Amphipteryngium adstringens* plant in CHO cells was performed in 96-well plates, using the MTT colorimetric method. This method is used as an indicator of mitochondrial function in living cells, therefore it may be used to measure cytotoxicity, cell viability and proliferation. The method relies on the ability of mitochondrial succinate dehydrogenase from metabolically active cells to reduce the MTT yellow color, to a dark blue colored compound (formazan). The presence of blue coloration allows determining the mitochondrial functionality of treated cells as viability parameter.

10,000 cells/well in 100 $\mu$l medium were seeded. After 24 h incubation at 37°C in a humid atmosphere with 5% $CO_2$ and cell monolayer in confluency, the cells were exposed for 12 and 24 hours to five extract dilutions. The treatment protocol was carried out in quadruplicate using three different controls: exposed to vehicle (ethanol), negative control (no vehicle or plant extract) and a positive control with 5% DMSO. The cellular viability was determined after the treatment period by MTT (3-(4,5-dimethylthiazol-2-yl)-2,5-diphenyltetrazole) metabolic reduction.

Viability was quantified according to the blue color intensity proportional to the amount of cells surviving the treatment. Treated group values were normalized to non-exposure control condition values (100% viability) and from this value the % of live cells in experimental conditions was obtained.

Toxicity experiments in vitro provided the following results.

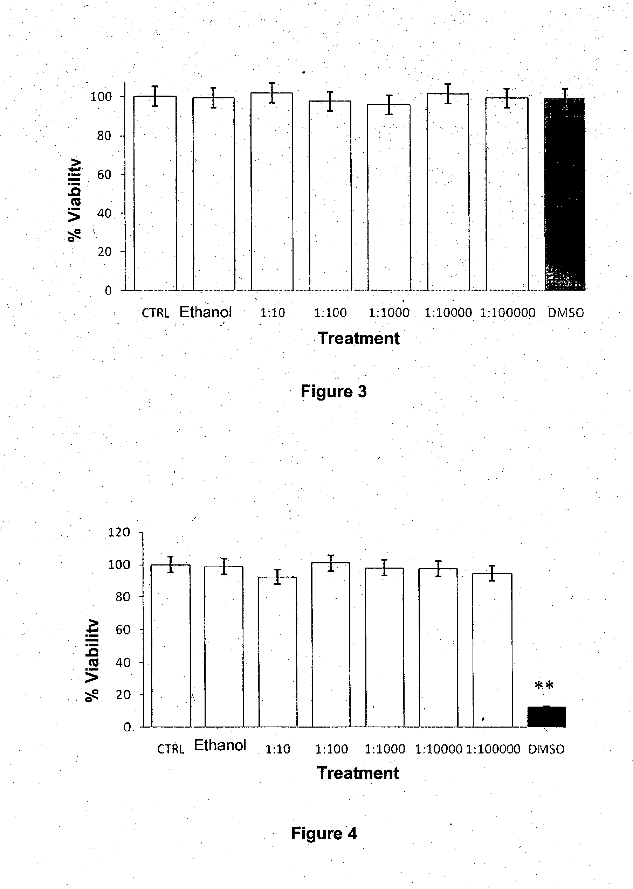

Figure 3 shows the cell viability results of a CHO cell line exposed for 12 hours to five different *Amphipteryngium adstringens* extract dilutions (1:10, 1:100, 1:1000, 1:10000, 1:100000) and none of the tested dilutions was toxic as they did not significantly reduce the viability.

It may be also seen that both vehicle treatment (ethanol) and DMSO used concentration as positive control (5%) did not affect CHO line viability after a 12-hour exposure period. Since the positive control did not reduce cell viability, it is difficult to make inferences about the results as cell model sensitivity is not demonstrated, then, exposure period was increased up to 24 hours under the same conditions.

Figure 4 shows the result observed by exposing a CHO cell line to *Amphipteryngium adstringens* extract serial dilutions for 24 hours.

Figure 4 demonstrates the sensitivity of the used cell model. DMSO concentration is observed to reduce significantly the cell viability after a 24-hour exposure period. Furthermore the data show that used ethanol concentration does not affect the CHO cell line viability, thus the observed effects in tested dilutions, are only due to *Amphipteryngium adstringens* exposure. After obtaining this result two higher concentration levels were chosen to provide low, medium and high dilution curves.

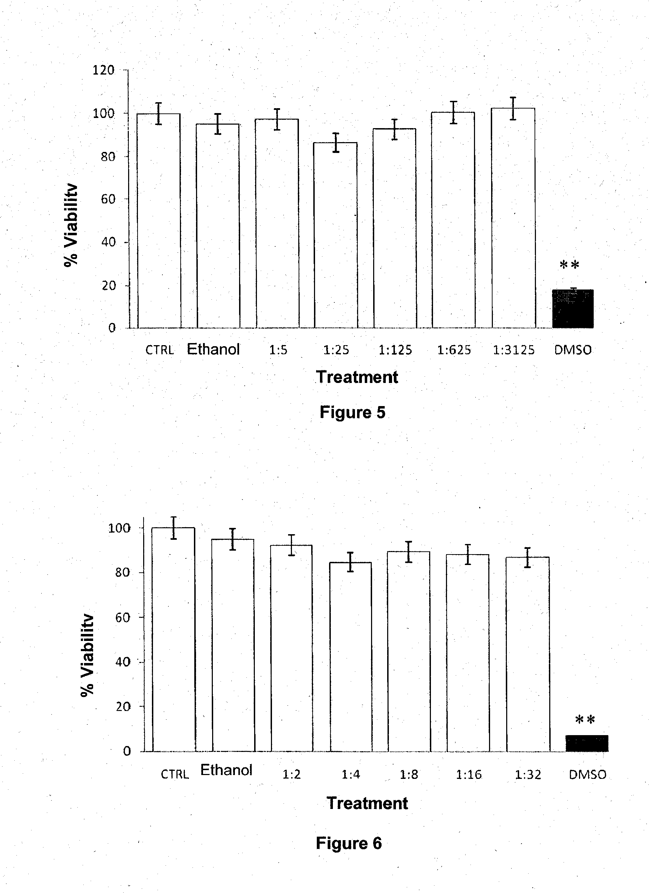

Figures 5 and 6 represent the mean and high curves respectively, and they show the cell viability results of a 24-hour exposure CHO cell line to five different *Amphipteryngium adstringens* extract dilutions. Figures 5 and 6 show that none of the tested dilutions significantly reduced the CHO cell line viability after a 24-hour exposure. Thus, the Cuachalalate extract provided by this invention resulted to be non-toxic.

**V. Quantification of active ingredients.**

The concentration of active ingredients (tannins) in the concentrated extract was quantified by the following methodology:

A suitable amount of the hydroalcoholic extract was weighed and placed into an Erlenmeyer flask with 100 ml distilled water and stirred until dissolved by magnetic stirring. The obtained solution is filtered and brought to a 250 ml volume. Then a 250 $\mu$l aliquot of the above solution was transferred in a 25 ml flask, and 20 ml of water and 1.25 ml of Folin-Denis reagent were added, thoroughly mixed, and 2.5 ml of $Na_2CO_3$ (35% w/w) were added after 5 minutes. The above mixture was brought to final volume with water, mixed and let stand, protected from light for 1 hour. After this period, a sample of above solution was taken to read absorbance at 700 nm using a solution prepared as a control according to disclosed procedure but not containing the sample to be determined. The tannin content (% w/w) was calculated using the following equation:

$$((Ct \times 250\ ml \times 100\ \mu g) \times 100)/Pm$$

Where:

Ct = Tannin concentration in $\mu$g/ml, interpolated on the standard curve.
Pm = Sample weight in $\mu$g.

200 mg of hydroalcoholic extract concentrate described in examples 1 and 2 were used for this determination. The samples were quantified by UV-visible spectroscopy at 700 nm using tannic acid as a reference standard. The standard curve, prepared with the same method, performs linearly up to a 8 pg/ml concentration with a linear correlation coefficient $R^2 > 0.9$. According to the procedure described herein, the tannin content in the hydroalcoholic concentrate extract of the invention, obtained from Cuachalalate bark and leaves, was 36% w/w.

**VI. Method for providing an EC topical formulation.**

An EC preparation is formulated with a solution, emulsion, gel, ointment, lotion, balm, cream, paste or dispersible solid that may be applied on the skin and form a thin layer thereon. Said preparation may also include other sunscreen (physical or chemical) agents which meet a wide range of protection (SPF at least 2), various additives such as isotonic agents such as sugars or polyalcohols, buffers, chelating agents, antioxidants and preservatives, in addition to emollients, humectants, lubricants, thickeners. Moreover, solutions containing the extract of the invention may also be formulated as a gel by addition of gelling agents.
An example of the use of the extract of the invention is its formulation as hydrogels using high molecular weight acrylic acid cross-linked polymers, such as Carbopol, because of its good bio-adhesive capacity.

**EXAMPLE 5**

Carbopol polymers are synthetic hydrophilic colloids, easily dispersable in water and available as a low oral toxicity, non-irritating, non-allergenic white powder, and with a molecular weight from 450,000 to 5,000,000. For purposes of the present invention, carbopol polymers that may be used to obtain hydrogels must provide a suitable viscosity compatible with the extract described in this invention. Carbopol 940 is selected among the different types of carbopol. The gelling agent is added to the hydrogel composition with the extract of the present invention previously dissolved in water at a concentration of 0.2 to 1% w/w, preferably between 0.5 and 1% w/w and in particular a concentration of 0.75% w/w.
In order to provide a proper dissolution of the extract of the invention and to allow its incorporation after hydrogel, polymers solvents such as, different molecular weight polyethylene glycols, e.g. polyethylene glycol 200, are used for mixing with the extract of the invention and the polymer at a ratio allowing a complete dilution of the extract, preferably in a 1:1 w/w. An alkaline neutralizing agent is then added to the group comprising triethanolamine, isopropylamine and diisopropylamine, preferably triethanolamine, to the hydrogel composition in an amount that allows proper adjustment of pH to physiological pH, e.g., pH 7.

**REFERENCES**

Autier P, Sunscreen abuse for intentional sun exposure, British Journal of Dermatology, 2009, Vol. 161, Issue s3, pp 40-45.
Autier P, Boniol M, Dore JF, Sunscreen use and increased duration of intentional sun exposure: still a burning issue. Int J Cancer., 2007 July 1, 121(1): 1-5.
Ganem Rondero, F.A. ET AL.: "Study of the photoprotective-cosmetic properties of Amphipterygium adstringens (Cuachalalate) extract", DATABASE KOSMET & IFSCC International conference. 1994 October; 3:686-703.
Hinneburg et al, 2005; Hinneburg I, Neubert R. Influence of Extraction Parameters on the Phytochemical Characteristics of Extracts from Buckwheat (Fagopyrum esculentum) Herb. J Agric Food Chem. 2005; 53:3-7.
Holick MF, The vitamin D deficiency pandemic and consequences for nonskeletal health: mechanisms of action, Mol Aspects Med., 2008 Dec; 29(6):361-8.
IARC Publications, Working group reports Vol 1, Exposure to Artificial UV Radiation and Skin Cancer, Jun 2005.
Kaminester LH, Current concepts, Photoprotection, Arch Fam Med, 1996 May; 5(5):289-95.
Kaminester LH, Current concepts. Photoprotection, Arch Fam Med, 1996 May;5(5):289-95.

Kitamura et al, 2004; Kitamura S, Shikazono N, Tanaka A. Transparent Testa 19is Involved in the Accumulation of Both Anthocyanins and Proanthocyanidins in Arabidopsis. The Plant Journal. 2004; 37: 104-14.

Reichrath J, Skin cancer prevention and UV-protection: how to avoid vitamin D-deficiency?, Br J Dermatol., 2009 Nov;161 Suppl 3:54-60.

Wang SQ, Dusza SW, Assessment of sunscreen knowledge: a pilot survey. Br J Dermatol., 2009 Nov; 161 Suppl 3:28-32.

WHO Fact Sheet No. 305, Ultraviolet radiation: solar radiation and human health, 2009.

## Claims

1. Natural composition with UV radiation absorption capacity, comprising a colloidal hydroalcoholic concentrate extract of *Amphipteryngium adstringens* bark and leaves and between 12 and 36% w/w of tannins measured as tannic acid concentration, for use in the prevention and treatment of induced skin lesions due to an overexposure to sun radiation.

2. A pharmaceutical composition comprising the composition for use according to claim 1, a high molecular weight acrylic acid polymer as gelling agent, an extract solvent polymer, an alkaline neutralization agent and water, in a pharmaceutically acceptable vehicle,
in which said pharmaceutical composition is a topical composition in the form of a solution, an emulsion, a gel, an ointment, a lotion, a balm, a cream, a paste or a dispersible solid.

## Patentansprüche

1. Natürliche Zusammensetzung mit UV-Strahlung-Absorptionsvermögen, welche einen kolloidalen hydroalkoholischen Konzentrat-Extrakt aus Rinde und Blättern von *Amphipteryngium adstringens* und zwischen 12 und 36 % w/w an Gerbstoffen umfasst, gemessen als Gerbsäurekonzentration, zur Verwendung bei der Vorbeugung und Behandlung von induzierten Hautschäden aufgrund einer übermäßigen Sonnenstrahlenaussetzung.

2. Pharmazeutische Zusammensetzung, welche die Zusammensetzung zur Verwendung gemäß Anspruch 1, ein hochmolekulares Acrylsäurepolymer als Geliermittel, ein Extrakt-Lösungsmittel-Polymer, ein alkalisches Neutralisationsmittel und Wasser in einem pharmazeutisch annehmbaren Träger umfasst,
wobei die pharmazeutische Zusammensetzung eine topische Zusammensetzung in Form einer Lösung, einer Emulsion, eines Gels, einer Salbe, einer Lotion, eines Balsams, einer Creme, einer Paste oder eines dispergierbaren Feststoffs ist.

## Revendications

1. Composition naturelle à capacité d'absorption de la radiation UV, comprenant un extrait concentré hydroalcoolique colloïdal d'écorce et de feuilles *d'Amphipteryngium adstringens* et entre 12 et 36% p/p de tannins mesurés en concentration d'acide tannique, pour une utilisation dans la prévention et le traitement des lésions cutanées induites par une surexposition aux rayonnements solaires.

2. Composition pharmaceutique comprenant la composition à utiliser selon la revendication 1, un polymère d'acide acrylique de poids moléculaire élevé comme agent gélifiant, un polymère solvant d'extrait, un agent de neutralisation alcalin et de l'eau, dans un véhicule pharmaceutiquement acceptable,
dans laquelle ladite composition pharmaceutique est une composition topique sous la forme d'une solution, d'une émulsion, d'un gel, d'une pommade, d'une lotion, d'un baume, d'une crème, d'une pâte ou d'un solide dispersible.

**Figure 1**

**Figure 2**

**Figure 3**

**Figure 4**

Figure 5

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5843421 A
- JP 10245393 B
- JP 10245394 B
- JP 11246413 B
- JP 11246415 B
- JP 2000103765 B
- US 20040022758 A
- JP 11209236 B
- JP 11209298 B
- JP 2000169497 B
- JP 11209261 B
- JP 2000247862 B
- JP 2004307443 A
- JP 2006062990 A
- JP 2000198715 A

### Non-patent literature cited in the description

- **AUTIER P.** Sunscreen abuse for intentional sun exposure. *British Journal of Dermatology,* 2009, vol. 161 (s3), 40-45
- **AUTIER P ; BONIOL M ; DORE JF.** Sunscreen use and increased duration of intentional sun exposure: still a burning issue. *Int J Cancer.,* 01 July 2007, vol. 121 (1), 1-5
- **GANEM RONDERO, F.A. et al.** Study of the photoprotective-cosmetic properties of Amphipterygium adstringens (Cuachalalate) extract. *DATABASE KO- SMET & IFSCC International conference,* October 1994, vol. 3, 686-703
- **HINNEBURG et al.** Hinneburg I, Neubert R. Influence of Extraction Parameters on the Phytochemical Characteristics of Extracts from Buckwheat (Fagopyrum esculentum) Herb. *J Agric Food Chem. 2005,* 2005, vol. 53, 3-7
- **HOLICK MF.** The vitamin D deficiency pandemic and consequences for nonskeletal health: mechanisms of action. *Mol Aspects Med.,* December 2008, vol. 29 (6), 361-8
- **KAMINESTER LH.** *Current concepts, Photoprotection, Arch Fam Med,* May 1996, vol. 5 (5), 289-95
- **KAMINESTER LH.** *Current concepts. Photoprotection, Arch Fam Med,* May 1996, vol. 5 (5), 289-95
- **KITAMURA ; 2004 et al.** Kitamura S, Shikazono N, Tanaka A. Transparent Testa 19is Involved in the Accumulation of Both Anthocyanins and Proanthocyanidins in Arabidopsis. *The Plant Journal,* 2004, vol. 37, 104-14
- **REICHRATH J.** Skin cancer prevention and UV-protection: how to avoid vitamin D-deficiency?. *Br J Dermatol.,* November 2009, vol. 161 (3), 54-60
- **WANG SQ ; DUSZA SW.** Assessment of sunscreen knowledge: a pilot survey. *Br J Dermatol.,* November 2009, vol. 161 (3), 28-32